# EUROPEAN PATENT APPLICATION

(11) **EP 4 721 794 A1**
(43) Date of publication of application: **08.04.2026**
(21) Application number: 24815678.8
(22) Date of filing: 27.03.2024
(51) Int. Cl.: A61M 15/00

(54) **INHALER**

(30) Priority: 31.05.2023 KR 20230069798
(71) Applicant: KT&G Corporation, Daedeok-gu Daejeon 34337 (KR)
(72) Inventor: KIM, Moonwon, Daejeon 34128 (KR); JUNG, Yongmi, Daejeon 34128 (KR); SHIN, Jun Won, Daejeon 34128 (KR)
(74) Representative: Ter Meer Steinmeister & Partner
(86) International application number: PCT/KR2024/003823
(87) International publication number: WO 2024/248299

(57) **Abstract**

An inhaler according to one embodiment comprises: a mouthpiece including a capsule containing functional materials; a housing having one end opened so that the mouthpiece is inserted therein; a piercing unit which is disposed inside the housing and which extends from the other end of the housing toward one end thereof; a sliding unit to which the mouthpiece is coupled and which slides inside the housing; and a stopper mounted on one side of the housing so as to limit the movement distance of the sliding unit, wherein an air inflow space can be ensured between the mouthpiece and the sliding unit.

## Description

### TECHNICAL FIELD

An inhaler is disclosed.

### BACKGROUND ART

A conventional dry powder inhaler is provided to transfer a pharmacological agent that is injected in a standard amount for a single dose into a body. The conventional dry powder inhaler has been used to treat patients with asthma and chronic obstructive pulmonary disease (COPD) and uses finely dried powders of 5 µm to 10 µm or less for efficient absorption of the agent into the body. Additionally, a hard capsule may be filled with a determined amount of drug to deliver a standard amount.

The above description is information the inventor(s) acquired during the course of conceiving the present disclosure, or already possessed at the time, and is not necessarily art publicly known before the present application was filed.

Prior Art: Korean Patent Application Publication No. 10-2006-0126490

### DISCLOSURE OF THE INVENTION

### TECHNICAL GOALS

A goal of an embodiment is providing an inhaler configured to inhale dry powder and including a structure for perforating and coupling a single-use or multi-use stick for inhaling dry powder.

The technical goals obtainable from the embodiments are not limited to the above-mentioned technical goals, and other unmentioned technical goals may be clearly understood from the following description by those having ordinary skill in the technical field to which the present disclosure pertains.

### TECHNICAL SOLUTIONS

An inhaler according to an embodiment to achieve the goal may include a mouthpiece including a capsule including a functional material, a housing of which an end is open to insert the mouthpiece therein, a piercing portion disposed in the housing and extending toward an end of the housing from the other end thereof, a sliding portion coupled to the mouthpiece and sliding inside the housing, and a stopper mounted on a side of the housing and limiting a movement distance of the sliding portion, wherein an air inlet space may be secured between the mouthpiece and the sliding portion.

According to an aspect, the sliding portion may include a moving member sliding inside the housing and having a cylindrical body, and a plurality of mounting members disposed apart from each other on a surface of the moving member in a circumferential direction and protruding toward the end of the housing, wherein the mounting member may be formed as a single-step bump of which a protruding height decreases toward a center thereof, and an end of the mouthpiece may be coupled to the mounting member to form the air inlet space.

According to an aspect, the inhaler may further include a plurality of guide grooves formed in a side surface of the moving member and each disposed between the two apart mounting members, wherein the guide groove may be coupled to a guide rail formed in the housing.

According to an aspect, the inhaler may further include a through hole penetrating the moving member in a same direction as a moving direction of the moving member, wherein, when the moving member slides in a direction from the end of the housing toward the other end of the housing, the piercing portion may pass through the through hole.

According to an aspect, in the through hole, a diameter of an opening adjacent to the other end of the housing may be greater than a diameter of an opening adjacent to the end of the housing.

According to an aspect, the inhaler may further include a spring of which an end is coupled to the sliding portion and the other end is coupled to the other end of the housing, wherein the spring may contract when the sliding portion is pressed and moves toward the other end of the housing, and when the sliding portion is not pressed, the spring may support the sliding portion to be positioned apart from the piercing portion toward the end of the housing.

According to an aspect, the stopper may be positioned in the housing so that an end of the stopper is positioned outside of the housing and the other end of the stopper is positioned further inward than an outer circumference of the sliding portion.

According to an aspect, an air inlet that penetrates the stopper may be formed on the stopper, and the air inlet may allow the air inlet space to communicate with the outside of the housing.

### EFFECTS OF THE INVENTION

According to an inhaler in an embodiment, dry powder may be inhaled, and a single-use or multi-use stick for inhaling dry powder may be perforated and may be coupled.

The effect of the inhaler according to the embodiment is not limited to the above-mentioned effect, and other unmentioned effects can be clearly understood from the following description by one of ordinary skill in the art.

### BRIEF DESCRIPTION OF DRAWINGS

FIG. 1 is a perspective view of an inhaler according to an embodiment.
FIG. 2 is a cross-sectional view of an inhaler according an embodiment.
FIG. 3 is a cross-sectional view of an inhaler in which a capsule is perforated by a piercing portion according to an embodiment.
FIG. 4 is a perspective view of a sliding portion of an inhaler according to an embodiment.
FIG. 5 is a cross-sectional view of a sliding portion of an inhaler according an embodiment.
FIG. 6 illustrates a stopper of an inhaler according to an embodiment.

The accompanying drawings illustrate preferred embodiments of the present invention, and are provided together with the detailed description for better understanding of the technical idea of the present invention. Therefore, the present invention should not be construed as being limited to the embodiments set forth in the drawings.

### BEST MODE FOR CARRYING OUT THE INVENTION

Hereinafter, the embodiments disclosed in the present specification will be described in detail with reference to the accompanying drawings. Regarding the reference numerals assigned to the components in the drawings, it should be noted that the same components will be designated by the same reference numerals, wherever possible, even though they are shown in different drawings. Furthermore, in the following description of the present embodiments, a detailed description of publicly known configurations or functions incorporated herein will be omitted when it is determined that the detailed description obscures the subject matters of the present embodiments.

Also, in the description of the components, terms such as first, second, A, B, (a), (b) or the like may be used herein when describing components of the present disclosure. These terms are used only for the purpose of discriminating one constituent element from another constituent element, and the nature, the sequences, or the orders of the constituent elements are not limited by the terms. It should be noted that if one component is described as being "connected," "coupled" or "joined" to another component, the former may be directly "connected," "coupled," and "joined" to the latter or "connected", "coupled", and "joined" to the latter via another component.

The same name may be used to describe an element included in the embodiments described above and an element having a common function. Unless otherwise mentioned, the description of one embodiment may be applicable to other embodiments. Thus, duplicated description is omitted for conciseness.

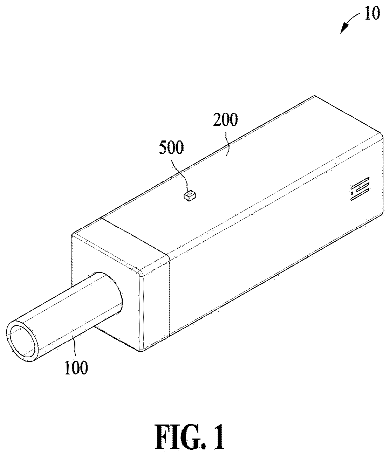

FIG. 1 is a perspective view of an inhaler 10 according to an embodiment.

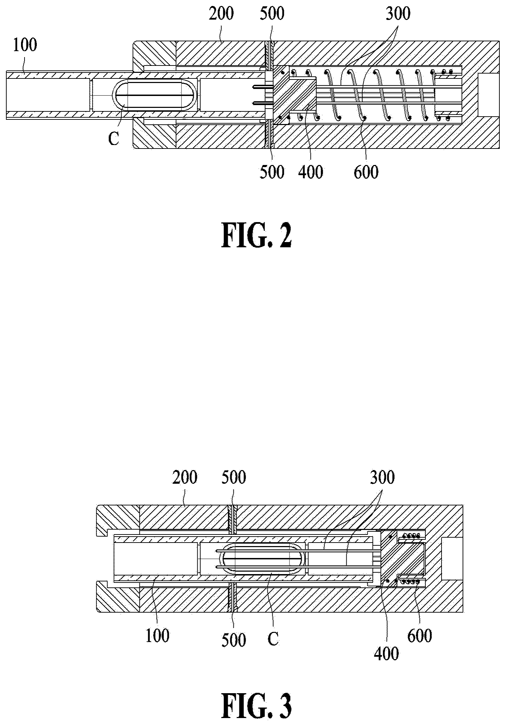

FIG. 2 is a cross-sectional view of the inhaler 10 according an embodiment.

FIG. 3 is a cross-sectional view of the inhaler 10 in which a capsule C is perforated by a piercing portion 300 according to an embodiment.

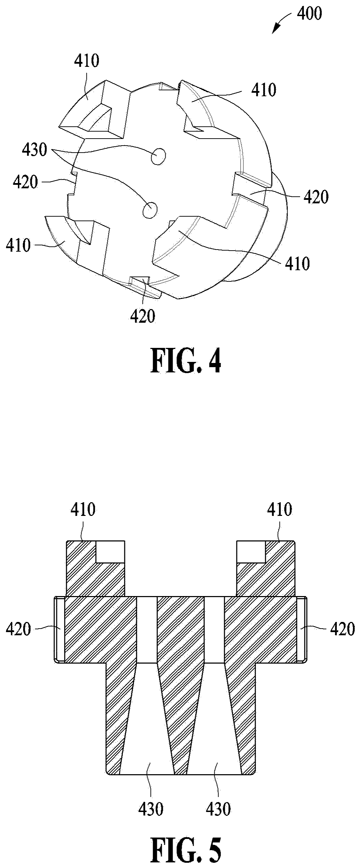

FIG. 4 is a perspective view of a sliding portion 400 of the inhaler 10 according to an embodiment.

FIG. 5 is a cross-sectional view of the sliding portion 400 of the inhaler 10 according an embodiment.

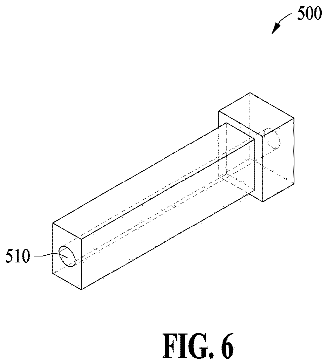

FIG. 6 illustrates a stopper 500 of the inhaler 10 according to an embodiment.

Referring to FIGS. 1 to 3, the inhaler 10 according to an embodiment may include a mouthpiece 100, a housing 200, the piercing portion 300, the sliding portion 400, and the stopper 500. Components of the inhaler 10 according to an embodiment may include materials, such as polylactic acid (PLA), acrylonitrile butadiene styrene copolymer (ABS), polycarbonate (PC), polypropylene (PP), etc., and may also include other biodegradable (eco-friendly) materials. Alternatively, the materials may be metal, such as aluminum.

The mouthpiece 100 may be formed in a cylindrical shape extending in a longitudinal direction, and an air inlet may be formed on a side surface thereof. The capsule C including a functional material may be included in the mouthpiece 100. The functional material may include, for example, nicotine, nicotine salts, caffeine, taurine, vitamins, and the like. The functional material may be contained in the capsule C, for example, as dry powder. In this case, the size of the dry powder may be approximately 5 µm to 10 µm, and menthol particles of 20 µm or more may be included in the dry powder. Alternatively, the size of the dry powder may be less than or equal to 5 µm. The dry powder may have or may not have a carrier. In this case, the size of the carrier may be 20 µm to 50 µm or more, and may be around 100 µm. An inhalation end portion of the mouthpiece 100 that a user contacts may include a mesh net, and a material of the mesh net may include PLA, the same material as the housing 200, or metal that is chemically safe and has no inhalation toxicity, such as stainless steel. The mouthpiece 100 may include a composite material of other biodegradable (eco-friendly) materials, paper, or cellulose acetate tow.

An end of the housing 200 may be open. The housing 200 may be provided in various shapes, such as a circle or square, that the user may easily grip. The mouthpiece 100 may be inserted into the housing 200 through one end. The housing 200 may be separated into an upper housing and a lower housing for a task, such as maintenance of components disposed in the housing 200 or removing internal contaminants. The upper housing and the lower housing may be separated from or coupled to each other through a screw structure formed on the upper housing.

The piercing portion 300 may be disposed in the housing 200. The piercing portion 300 may be disposed on the other end of the housing 200. The piercing portion 300 may extend toward one end. The piercing portion 300 may perforate the capsule C. The piercing portion 300 may be coupled as a replaceable structure from the other end of the housing 200 for separate maintenance. For example, the piercing portion 300 may be provided as a needle. The piercing portion 300 may be one needle, but preferably, two needles may be disposed in parallel. The two needles may have the same or different lengths, and the diameters of needles having different lengths may vary. The diameter of the needle may be provided as, for example, 0.5, 0.7, 0.9, or 1.0 mm, and preferably, may be provided as 0.7 mm.

The sliding portion 400 may be disposed to slide in the housing 200. In this case, an uneven structure may be formed inside the housing 200 to prevent the sliding portion 400 from deviating from a path. For example, a guide rail movably coupled to the sliding portion 400 may be formed inside the housing 200. The structure may allow the sliding portion 400 to move parallel to the piercing portion 300 when the mouthpiece 100 and the sliding portion 400 descend to perforate the capsule C. The end of the mouthpiece 100 may be coupled to the sliding portion 400. When the sliding portion 400 slides, the mouthpiece 100 may also move together. Additionally, when the mouthpiece 100 is coupled to the sliding portion 400, an air inlet space may be formed between the mouthpiece 100 and the sliding portion 400.

The stopper 500 may be mounted on the side surface of the housing. The stopper 500 may limit a movement distance of the sliding portion 400.

Referring to FIGS. 2 and 3, the inhaler 10 according to an embodiment may further include a spring 600.

An end of the spring 600 may be coupled to the sliding portion 400, and the other end of the spring 600 may be coupled to the other end of the housing 200. For example, the piercing portion 300 including two needles may be disposed at a distance less than the diameter of the spring 600 and may be disposed to be surrounded by the spring 600.

Referring to FIG. 2, when the mouthpiece 100 or the sliding portion 400 is not pressed by an external force, the spring 600 may support the sliding portion 400 so that the sliding portion 400 is positioned apart from the piercing portion 300 toward one end of the housing 200.

Referring to FIG. 3, when the mouthpiece 100 or the sliding portion 400 is pressed and moves toward the other end of the housing 200, the spring 600 may contract. Accordingly, as the piercing portion 300 penetrates the sliding portion 400 and the mouthpiece 100, the capsule C accommodated in the mouthpiece 100 may be perforated.

A functional material, for example, in the form of dry powder may be contained in the capsule C. As described above, as the capsule C is perforated by the piercing portion 300, the functional material in the capsule C may be inhaled by the user. In addition, since the user inhales the functional material while the capsule C is coupled to the mouthpiece 100, the inhalation resistance may increase.

Referring to FIG. 4, the sliding portion 400 may include a moving member, a mounting member 410, a guide groove 420, and a through hole 430.

The moving member may have a cylindrical body. The mounting member 410 may be formed on one surface of the moving member 410, and a protruding member that cylindrically protrudes toward the other end of the housing 200 may be formed. The diameter of the protruding member may be formed less than the diameter of the moving member. An end of the spring 600 may be coupled to the protruding member. Accordingly, the moving member may efficiently compress the spring 600. In this case, the wire diameter of the spring 600 may be between 0.5 mm and 1.0 mm.

A plurality of mounting members 410 may be provided and may be disposed on one surface of the moving member. In this case, the plurality of mounting members 410 may be spaced apart from each other in a circumferential direction of the moving member. The mounting member 410 may protrude from the one surface of the moving member toward the one end of the housing 200.

The mounting member 410 may be formed as, for example, a two-step bump. As shown in FIG. 4, the mounting member 410 may be formed as a single-step bump of which a protruding height decreases from the one surface of the moving member toward the center of the mounting member 410. The end of the mouthpiece 100 may be mounted on the mounting member 410. Due to the single-step bump shape of the mounting member 410, the mouthpiece 100 may not be in contact with the one surface of the moving member, and an air inlet space may be secured between the one surface and the mouthpiece 100. Accordingly, the inhaler 10 according to an embodiment may enable smooth airflow, and the user may easily inhale the functional material in the capsule C while the mouthpiece 100 is coupled to the mounting member 410.

The guide groove 420 may be formed in a side surface of the moving member. A plurality of guide grooves 420 may be provided, and each of the guide grooves 420 may be disposed between two apart mounting members 410.

A guide rail may be formed inside the housing 200 in a longitudinal direction of the housing 200. In the sliding portion 400, the guide rail may be coupled to the guide groove 420. Accordingly, when the sliding portion 400 moves inside the housing 200, the sliding portion 400 may move along the guide rail and may be prevented from rotating when moving.

Referring to FIGS. 4 and 5, the through hole 430 may be formed to penetrate the moving member in the same direction as the moving direction of the moving member. When the moving member slides in a direction from one end of the housing 200 toward the other end of the housing 200, the piercing portion 300 may pass through the through hole 430.

Referring to FIG. 5, the through hole 430 may be formed in a flared shape. For example, in the through hole 430, a diameter of an opening adjacent to the other end of the housing 200 may be greater than a diameter of an opening adjacent to the one end of the housing 200. The shape of the through hole 430 described above may allow the piercing portion 300 to smoothly pass through the through hole 430 so that the piercing portion 300 may reach the capsule C.

Referring to FIGS. 2 and 3, the stopper 500 may be inserted into the side surface of the housing 200 in a lateral direction of the housing 200. The stopper 500 may be inserted so that an end of the stopper 500 may be positioned outside of the housing 200 and the other end thereof may be positioned on the sliding portion 400 in the housing 200. In this case, the other end of the stopper 500 may be inserted into the housing 200 so that the other end of the stopper 500 may be positioned further inward than the outer circumference of the moving member, as shown in FIGS. 2 and 3.

Referring to FIG. 6, an air inlet 510 that penetrates the inside of the stopper 500 may be formed on the stopper 500. The air inlet 510 may allow the air inlet space between the mouthpiece 100 and the sliding portion 400 described above to communicate with the outside of the housing 200. Accordingly, the inhaler 10 according to an embodiment may enable smooth airflow from the outside to the air inlet space, and the user may easily inhale the functional material in the capsule C. A plurality of stoppers 500 may be provided for smooth airflow in the inhaler 10. For example, the plurality of stoppers 500 may be disposed in a point-symmetric arrangement. The stopper 500 may be coupled to the inhaler 10 by using a force fit or the like.

As described above, in the inhaler 10 according to an embodiment, as an end of the mouthpiece 100 is fixed to the mounting member 410 of the sliding portion 400 to secure the air inlet space between the mouthpiece 100 and the sliding portion 400, air may smoothly enter from the end of the mouthpiece 100 and the side surface of the mouthpiece 100.

In addition, the inhaler 10 according to an embodiment may be inhaled while the housing 200 is coupled to the mouthpiece 100 to ensure inhalation resistance similarity, and a level of the inhalation resistance when the mouthpiece 100 is coupled may be 30 mmH₂O to 50 mmH₂O.

While the embodiments of the present disclosure have been described above with reference to specific components, and limited embodiments and drawings, the above descriptions are merely for better understanding of the present disclosure, and it will be apparent to one of ordinary skill in the art that various changes in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents. Accordingly, the scope of the present disclosure is defined not by the detailed description, but by the claims and their equivalents, and all variations within the scope of the claims and their equivalents are to be construed as being included in the disclosure.

## Claims

1. An inhaler comprising:
a mouthpiece comprising a capsule comprising a functional material;
a housing of which an end is open to insert the mouthpiece therein;
a piercing portion disposed in the housing and extending toward an end of the housing from the other end thereof;
a sliding portion coupled to the mouthpiece and sliding inside the housing; and
a stopper mounted on a side of the housing and limiting a movement distance of the sliding portion,
wherein an air inlet space is secured between the mouthpiece and the sliding portion.

2. The inhaler of claim 1,
wherein the sliding portion comprises:
a moving member sliding inside the housing and having a cylindrical body; and
a plurality of mounting members disposed apart from each other on a surface of the moving member in a circumferential direction and protruding toward the end of the housing,
wherein the mounting member is formed as a single-step bump of which a protruding height decreases toward a center thereof, and an end of the mouthpiece is coupled to the mounting member to form the air inlet space.

3. The inhaler of claim 2, further comprising:
a plurality of guide grooves formed in a side surface of the moving member and each disposed between the two apart mounting members,
wherein the guide groove is coupled to a guide rail formed in the housing.

4. The inhaler of claim 2, further comprising:
a through hole penetrating the moving member in a same direction as a moving direction of the moving member,
wherein, when the moving member slides in a direction from the end of the housing toward the other end of the housing, the piercing portion passes through the through hole.

5. The inhaler of claim 4,
wherein, in the through hole, a diameter of an opening adjacent to the other end of the housing is greater than a diameter of an opening adjacent to the end of the housing.

6. The inhaler of claim 1, further comprising:
a spring of which an end is coupled to the sliding portion and the other end is coupled to the other end of the housing,
wherein the spring contracts when the sliding portion is pressed and moves toward the other end of the housing, and
when the sliding portion is not pressed, the spring supports the sliding portion to be positioned apart from the piercing portion toward the end of the housing.

7. The inhaler of claim 1,
wherein the stopper is positioned in the housing so that an end of the stopper is positioned outside of the housing and the other end of the stopper is positioned further inward than an outer circumference of the sliding portion.

8. The inhaler of claim 7,
wherein an air inlet that penetrates the stopper is formed on the stopper, and
the air inlet allows the air inlet space to communicate with the outside of the housing.
